# NOUVEAU FASCICULE DE BREVET EUROPEEN

(11) **EP 1 138 661 B2**
(45) Date de publication et mention de la décision concernant l'opposition: **20.11.2013**
(45) Mention de la délivrance du brevet: 28.04.2004
(21) Numéro de dépôt: 01400772.8
(22) Date de dépôt: 26.03.2001
(51) Int. Cl.: C07C 31/26, A61K 47/10, A61K 9/14

(54) **Mannitol pulvérulent et son procédé de préparation**
Pulverisiertes Mannit und Verfahren zu deren Herstellung
Pulverised mannitol and process for its preparation

(30) Priorité: 29.03.2000 FR 0003954
(43) Date de publication de la demande: 04.10.2001
(73) Titulaire: Roquette Frères, 62136 Lestrem (FR)
(72) Inventeur: Labergerie, Erik, 62136 Lestrem (FR); Lefevre, Philippe, 59400 Merville (FR); Lis, José, 59253 La Gorgue (FR)
(74) Mandataire: Cabinet Plasseraud

(56) Documents cités:
- EP-B1- 0 645 096
- WO-A1-97//38960
- FR-A- 2 571 045
- FR-A- 2 710 637
- E-mail v. 27.07.04
- Ergebnistabelle Partikelgrößenmessung COULTER LS 100 und LS 230 mit verschiedenen Dispergiereinheiten (naß u. trocken)
- E-mail HOSOKAWA v. 09.07.2004
- Versuchskontrolldaten, Kundenanfrage, Lieferscheine und Analysenzertifikat zu Versuch 42
- Versuchskontrolldaten, Kundenanfrage, Lieferscheine und Analysenzertifikat zu Versuch 39
- Versuchsauswertung
- Messwerte Rütteldichte und Fließwert Versuch 39
- Messwerte Rütteldichte und Fließwert Versuch 42
- J. SWARBRICK ET AL: '"Encyclopedia of Pharmaceutical Technology', vol. 16, 1997, M. DEKKER, NEW YORK pages 363 - 387
- 'Europäisches Arzneibuch, 6. Ausgabe, Grundwerk 2008(in Englisch und Deutsch).', 2008, DEUTSCHER APOTHEKER VERLAG, STUTTGART
- J. SWARBRICK ET AL: '"Encyclopedia of Pharmaceutical Technology"', vol. 4, 1991, M. DEKKER, NEW YORK pages 60 - 61
- Eidestattliche Versicherung James Easson
- Eidestattliche Versicherung Dr. G. Koch
- Merck interne Arbeitsanweisung zum Umgang mit Rückstellmustern
- A. BURGER ET AL: 'Energy/Temperature Diagram and Compression Behavior of the Polymorphs of D-Mannitol' JOURNAL OF PHARMACEUTICAL SCIENCE vol. 89, no. 4, Avril 2000, pages 457 - 468

## Description

La présente invention a pour objet un mannitol pulvérulent de fine granulométrie, de densité élevée et d'excellente aptitude à l'écoulement, présentant par ailleurs une richesse élevée en mannitol et une vitesse rapide de dissolution dans l'eau.

L'invention concerne également un procédé de fabrication dudit mannitol, ainsi que son utilisation dans les domaines pharmaceutique et alimentaire.

Les industries pharmaceutique et alimentaire sont consommatrices de quantités importantes de polyols pulvérulents, en tant qu'excipients, édulcorants massiques ou supports d'additifs. Il s'agit plus précisément du sorbitol, du xylitol, du mannitol et du maltitol.

Le sorbitol présente l'avantage d'être parmi ces trois polyols le produit le moins cher, ce qui explique sa grande fréquence d'emploi. Cependant, sa forte hygroscopicité conduit, dès lors qu'une reprise en eau est intervenue, à un produit dont l'écoulement est difficile, voire impossible.

Pour éviter ce problème, on sélectionne un sorbitol de granulométrie plus grossière mais alors les temps de dissolution dans l'eau deviennent en général trop longs. De plus, le caractère hygroscopique élevé du sorbitol rend dans tous les cas l'utilisation de ce polyol rédhibitoire quand il est associé à des principes actifs ou des ingrédients très sensibles à l'eau.

Le xylitol, quant à lui, n'est guère utilisé comme excipient car il présente l'inconvénient de prendre en masse dans des conditions normales d'humidité, et ceci encore plus facilement que le sorbitol.

Le mannitol, en raison de sa faible hygroscopicité, pourrait constituer un excellent excipient, étant donné qu'il est compatible avec la plupart des principes actifs, mais malheureusement, le produit obtenu par cristallisation dans l'eau à partir d'une solution sursaturée présente des propriétés d'écoulement médiocre.

En effet, le mannitol cristallisé présente une friabilité excessive, ce qui conduit à la formation de fines particules qui nuisent particulièrement à ses propriétés d'écoulement.

Le mannitol obtenu par cristallisation dans l'eau présente par ailleurs, en raison de sa structure cristalline compacte, une mauvaise aptitude à la dissolution. Cette faible vitesse de solubilisation, bien que pouvant être avantageuse pour certaines applications particulières, est dans les cas auxquels on s'intéresse ici toujours considérée comme un inconvénient majeur faisant obstacle à son emploi.

D'autres formes pulvérulentes de mannitol ainsi que les moyens d'obtention de celles-ci sont décrits dans la littérature.

Par exemple, le brevet US 3,341,415 a trait à une méthode de préparation d'un excipient pharmaceutique contenant au moins 20 % en poids de mannitol et un sucre additionnel choisi parmi le lactose, le saccharose, l'érythritol, le galactose et le sorbitol. Cependant, le procédé décrit est très délicat à mettre en oeuvre à une échelle industrielle. Le produit obtenu est en outre très hygroscopique, très compact et très difficile à solubiliser dans l'eau.

La demande de brevet JP 61.85330 est relative à un procédé de préparation d'excipients, caractérisé par le fait qu'il consiste à sécher du D-mannitol par nébulisation. Cependant, il apparaît que les produits ainsi obtenus contiennent plus de 50 % de particules de taille inférieure à 75 µm, ce qui est préjudiciable à un écoulement correct du produit.

Le brevet US 3,145,146 décrit un procédé de modification des caractéristiques physiques du mannitol grâce à un séchage par pulvérisation, et le produit ainsi obtenu.

On obtient ainsi une poudre dont la granulométrie est comprise entre 5 et 100 µm. Cependant, ce procédé consiste à introduire un liant, qui peut être une paraffine, une gomme ou un dérivé de cellulose, avant l'étape d'atomisation subséquente. De plus, encore au moins 50 % des particules de la poudre ont encore une taille inférieure à 75 µm, ce qui est loin d'être idéal pour obtenir un bon écoulement.

De tout ce qui précède, il résulte qu'il existe un besoin non satisfait de disposer d'un mannitol en tant qu'excipient, qui présente une fine granulométrie, une densité élevée et une excellente aptitude à l'écoulement, ces caractéristiques pouvant être avantageusement associées à une richesse élevée en mannitol et une vitesse rapide de dissolution dans l'eau.

En outre, pour que cet excipient soit utilisé de manière préférentielle en tant que poudre de remplissage de gélules, il est établi qu'il est nécessaire de disposer d'un produit qui soit compatible avec le principe actif auquel il est associé, que ses propriétés d'écoulement libre, d'homogénéité de mélange, de profil de dissolution et de densité tassée soit conforme à l'application souhaitée.

Pour obtenir un tel excipient possédant l'ensemble des caractéristiques fonctionnelles énoncées ci-dessus, la société Demanderesse a constaté que, contre toute attente, il convenait de choisir parmi les polyols un mannitol cristallin pur et de modifier ses caractéristiques physiques en employant un procédé approprié de sorte qu'il présente simultanément une fine granulométrie, une densité élevée et une excellente aptitude à l'écoulement, et par ailleurs une richesse élevée en mannitol et une vitesse rapide de dissolution dans l'eau.

La société Demanderesse a donc eu le mérite de concilier tous ces objectifs réputés jusqu'alors inconciliables, en imaginant et élaborant, au prix de nombreuses recherches, un nouveau mannitol pulvérulent.

L'invention se rapporte donc à un mannitol pulvérulent caractérisé en ce qu'il présente :
- un diamètre moyen compris entre 60 et 200 µm, de préférence compris entre 80 et 180 µm,
- une densité tassée, déterminée selon un test A, comprise entre 0,65 et 0,85 g/ml, de préférence comprise entre 0,7 et 0,8 g/ml,
- une note d'écoulement d'au moins 60, de préférence comprise entre 60 et 90.

Le mannitol pulvérulent conforme à l'invention présente un diamètre moyen compris entre 60 et 200 µm , de préférence compris entre 80 et 180 µm. Ces valeurs sont déterminées sur un granulomètre LASER LS de marque COULTER®, par la détermination de la répartition volumique en taille des particules de mannitol pulvérulent.

La taille des particules constitutifs du mannitol pulvérulent conforme à l'invention permet alors de lui conserver un diamètre moyen en rapport avec celui de la majorité des principes actifs, et ainsi d'obtenir des mélanges principes actifs / mannitol homogènes par équivalence granulométrique.

En effet, il est établi en toute généralité qu'une taille élevée des cristaux de principes actifs nuirait à leur vitesse de solubilisation. Un diamètre moyen compris entre 80 et 200 µm est donc recommandé.

Le mannitol pulvérulent conforme à l'invention peut également être caractérisé par sa densité tassée.

La détermination de la densité tassée est réalisée selon la méthode préconisée dans le mode d'emploi du testeur de poudre HOSOKAWA de type P.T.N.

Dans ces conditions, le mannitol pulvérulent conforme à l'invention présente une densité tassée élevée, c'est à dire comprise entre 0,65 et 0,85 g/ml, de préférence comprise entre 0,7 et 0,8 g/ml.

Cette valeur élevée en densité confère au mannitol pulvérulent conforme à l'invention des propriétés particulièrement adaptées à son utilisation comme agent de remplissage des gélules de petite taille en pharmacie, taille qui sera plus facilement acceptée par les patients.

Par ailleurs, on peut également caractériser le mannitol pulvérulent conforme à l'invention par son aptitude à l'écoulement, cette propriété convenant particulièrement lorsque ledit mannitol est utilisé pour remplir des gélules.

L'aptitude à l'écoulement dudit mannitol est évaluée en utilisant l'appareil POWDER TESTER commercialisé par la société HOSOKAWA. Cet appareil permet de mesurer, dans des conditions standardisées et reproductibles, l'aptitude à l'écoulement d'une poudre et de calculer une note d'écoulement, encore appelée indice de Carr.

Le mannitol pulvérulent conforme à l'invention présente une note d'écoulement excellente, généralement d'au moins 60, de préférence comprise entre 60 et 90.

Cette valeur est généralement bien supérieure à celle des poudres de mannitol cristallin de l'art antérieur et est équivalente aux mannitol poudres obtenus par des procédés d'extrusion ou d'atomisation.

Cependant, les produits obtenus par extrusion présentent habituellement une forte granulométrie de diamètre moyen compris entre 250 et 600 µm, et ceux obtenus par atomisation possèdent classiquement une faible densité tassée, inférieure à 0,6 g/ml, ce qui rend ces deux catégories de produits particulièrement peu adaptées aux domaines d'applications recherchés.

Le mannitol pulvérulent conforme à l'invention est caractérisé en ce qu'il présente également une richesse en mannitol au moins égale à 96 % en poids, de préférence au moins égale à 98 % en poids.

Du point de vue de sa composition chimique, le mannitol pulvérulent conforme à l'invention est donc relativement pur.

Il est donc surprenant et inattendu qu'un mannitol pulvérulent, qui présente une telle aptitude à l'écoulement, déjà pour une fine granulométrie et une telle densité tassée, présente conjointement une telle pureté chimique.

En effet, les seuls mannitol pulvérulents présentant une bonne aptitude à l'écoulement, à la connaissance de la société Demanderesse, comportent, comme il a été dit plus haut, des liants tels que de la paraffine, des gommes ou dérivés de la cellulose.

Enfin, le mannitol pulvérulent conforme à l'invention est caractérisé par sa vitesse rapide de dissolution dans l'eau, vitesse mesurée selon un test B mis au point par la société Demanderesse.

Pour mesurer la vitesse de dissolution dans l'eau selon le test B, on introduit dans 150 ml d'eau déminéralisée et dégazée, maintenue à 20 °C et soumise à une agitation à 200 t/min, 5 g exactement du produit à tester.

Le temps de dissolution correspond au temps nécessaire, après introduction du produit, pour obtenir une parfaite limpidité visuelle de la suspension ainsi préparée.

Dans ces conditions, le mannitol pulvérulent conforme à l'invention possède une vitesse de dissolution rapide, c'est à dire comprise entre 20 et 60 secondes. Ces temps de dissolution sont généralement bien adaptés aux applications visées.

Le mannitol pulvérulent conforme à l'invention est obtenu en procédant à une étape de granulation d'un mannitol poudre par voie humide à l'aide d'un liant, puis à une étape de maturation par séchage du mannitol pulvérulent ainsi obtenu.

Pour obtenir un mannitol pulvérulent conforme à l'invention possédant les caractéristiques fonctionnelles énoncées, la société Demanderesse a constaté qu'il convenait de choisir comme mannitol de départ un mannitol poudre, obtenu par cristallisation dans l'eau ou dans un autre solvant comme l'alcool.

La granulométrie dudit mannitol poudre de départ ne constitùe pas en soi un facteur limitant pour produire un mannitol pulvérulent conforme à l'invention.

Le liant est quant à lui constitué d'eau ou d'un sirop de mannitol d'une matière sèche au plus égale à 50 %, de préférence compris entre 20 et 40 %, ou encore de vapeur d'eau, comme il sera exemplifié ci-après.

De manière surprenante et inattendue, la société Demanderesse a constaté que la granulation d'un mannitol poudre par voie humide à l'aide d'un liant permet de préparer avec un haut rendement un produit conforme à l'invention sur le plan de sa granulométrie, de sa densité et de son aptitude à l'écoulement.

En effet, les procédés décrits antérieurement ne permettent pas d'obtenir l'ensemble des caractéristiques souhaitées.

Pour procéder à la granulation, on peut employer par exemple un mélangeur-granulateur continu de type FLEXOMIX vertical commercialisé par la société HOSOKAWA SCHUGI, ou de type CB horizontal commercialisé par la société LÖDIGE dans lequel on introduit, via un doseur pondéral, le mannitol poudre de départ à granuler en continu et via un doseur volumétrique, le liant (de l'eau, de la vapeur d'eau ou la solution de mannitol) en continu. La granulation peut également s'effectuer dans une tour d'atomisation, ou dans un granulateur à lit fluidisé.

De manière préférentielle, on choisit d'utiliser un mélangeur-granulateur continu de type FLEXOMIX vertical HOSOKAWA SCHUGI. La poudre de mannitol de départ et le liant sont très intimement mélangés dans le mélangeur-granulateur équipé d'un axe avec couteaux disposés en pales, et d'un système de pulvérisation de liquides par buses d'injection.

Dans un mode préférentiel du procédé, la bonne dispersion des constituants et l'agglomération des particules de la poudre de mannitol de départ sont réalisées par agitation à grande vitesse, c'est-à-dire d'une valeur au moins égale à 2000 rpm, de préférence au moins égale à 3000 rpm. A la sortie du mélangeur-granulateur, les granulés formés sont déchargés en continu sur un séchoir. Le déchargement se fait préférentiellement par gravité dans le cas dudit granulateur vertical, et par poussée, via l'axe des couteaux rotatifs, si le granulateur horizontal est utilisé.

Cette deuxième étape de séchage en sortie du mélangeur-granulateur permet d'éliminer l'eau provenant du liant et de cristalliser la matière sèche provenant du liant, dans le cas où une solution de mannitol a été mise en oeuvre, de manière à ce que la cristallisation se produise après l'étape préalable de granulation. Le séchoir peut être par exemple un séchoir à lit fluidisé ou un tambour maturateur rotatif.

Le mannitol pulvérulent conforme à l'invention est obtenu après refroidissement et éventuellement tamisage. Dans ce cas, les fines particules peuvent être directement recyclées en tête de granulation, et les grosses particules être broyées et recyclées en tête de tamisage ou en tête de granulation.

D'autres caractéristiques et avantages de l'invention apparaîtront à la lecture des exemples qui suivent. Ils ne sont toutefois donnés ici qu'à titre illustratif et non limitatif.

### EXEMPLE 1

On alimente un mélangeur-granulateur FLEXOMIX vertical SCHUGI en continu via un doseur poudre, à un débit de 500 kg/h, avec un mannitol cristallin, fabriqué par cristallisation.

D'autre part, on alimente le mélangeur-granulateur en continu avec une solution de mannitol à 50 % en poids à 80°C et à un débit de 60 à 70 l/h, via une buse de pulvérisation. L'axe rotatif à couteaux est préalablement réglé à la vitesse de 3000 tpm.

La poudre granulée humide en sortie du mélangeur-granulateur tombe en continu, par gravité, dans un séchoir - maturateur à lit fluidisé SCHUGI à 4 compartiments. Dans les 3 premiers compartiments, le produit granulé est séché par de l'air à 125-150°C, puis il est refroidi par de l'air à 30°C dans le dernier compartiment.

Le produit granulé, séché et refroidi est ensuite tamisé en continu sur un tamis rotatif équipé d'une toile métallique de 740 µm.

Le mannitol cristallin de départ A et 12 mannitol pulvérulent B conforme à l'invention préparé avec une solution de mannitol comme liant, présentent les caractéristiques rassemblées dans le tableau I suivant.

**Tableau I**

| Paramètres | A | B |
|---|---|---|
| Diamètre moyen laser (µm) | 62 | 106 |
| Densité tassée (g/ml) | 0,79 | 0,71 |
| Note d'écoulement (valeur/100) | 40 | 67 |
| Richesse en mannitol (% en poids) | > 98 | > 98 |
| Vitesse de dissolution dans l'eau (s) | 83 | 36 |

### EXEMPLE 2

On alimente un mélangeur-granulateur FLEXOMIX vertical SCHUGI en continu via un doseur poudre, à un débit de 500 kg/h, avec un mannitol cristallin, fabriqué par cristallisation.

D'autre part, on alimente le mélangeur-granulateur en continu avec une solution de mannitol à 40 % en poids à 90°C et à un débit de 90 l/h, via une buse de pulvérisation. L'axe rotatif à couteaux est préalablement réglé à la vitesse de 3000 tpm.

La poudre granulée humide en sortie du mélangeur-granulateur tombe en continu, par gravité, dans un séchoir - maturateur à lit fluidisé SCHUGI à 4 compartiments. Dans les 3 premiers compartiments, le produit granulé est séché par de l'air à 125-150°C, puis il est refroidi par de l'air à 30°C dans le dernier compartiment.

Le produit granulé, séché et refroidi est ensuite tamisé en continu sur un tamis rotatif équipé d'une toile métallique de 740 µm.

Le mannitol cristallin de départ A et le mannitol pulvérulent C conforme à l'invention préparé avec une solution de mannitol comme liant, présentent les caractéristiques rassemblées dans le tableau II suivant.

**Tableau II**

| Paramètres | A | C |
|---|---|---|
| Diamètre moyen laser (µm) | 62 | 126 |
| Densité tassée (g/ml) | 0,79 | 0,71 |
| Note d'écoulement (valeur/100) | 40 | 65 |
| Richesse en mannitol (% en poids) | > 98 | > 98 |
| Vitesse de dissolution dans l'eau (s) | 83 | 43 |

### EXEMPLE 3

On alimente un mélangeur-granulateur FLEXOMIX vertical SCHUGI en continu via un doseur poudre, à un débit de 450 kg/h, avec un mannitol cristallin, fabriqué par cristallisation.

D'autre part, on alimente le mélangeur-granulateur en continu avec de la vapeur d'eau à une pression de 1,3 bars à une température de 107°C à un débit de 40 kg/h, via une buse de pulvérisation. L'axe rotatif à couteaux est préalablement réglé à la vitesse de 3000 tpm.

La poudre granulée humide en sortie du mélangeur-granulateur tombe en continu, par gravité, dans un séchoir - maturateur à lit fluidisé SCHUGI à 4 compartiments. Dans les 3 premiers compartiments, le produit granulé est séché par de l'air à 150°C, puis il est refroidi par de l'air à 30°C dans le dernier compartiment.

Le produit granulé, séché et refroidi est ensuite tamisé en continu sur un tamis rotatif équipé d'une toile métallique de 740 µm.

Le mannitol cristallin de départ A et le mannitol pulvérulent D conforme à l'invention préparé avec de la vapeur d'eau comme liant, présentent les caractéristiques rassemblées dans le tableau III suivant.

**Tableau III**

| Paramètres | A | D |
|---|---|---|
| Diamètre moyen laser (µm) | 62 | 101 |
| Densité tassée (g/ml) | 0,79 | 0,70 |
| Note d'écoulement (valeur/100) | 40 | 67 |
| Richesse en mannitol (% en poids) | > 98 | > 98 |
| Vitesse de dissolution dans l'eau (s) | 83 | 33 |

### EXEMPLE 4

On alimente un mélangeur-granulateur FLEXOMIX vertical SCHUGI en continu via un doseur poudre, à un débit de 800 kg/h, avec un mannitol cristallin, fabriqué par cristallisation.

D'autre part, on alimente le mélangeur-granulateur en continu avec une solution de mannitol à 20 % en poids à une température de 50°C à un débit de 75 l/h, via une buse de pulvérisation. L'axe rotatif à couteaux est préalablement réglé à la vitesse de 3650 tpm.

La poudre granulée humide en sortie du mélangeur-granulateur tombe en continu, par gravité, dans un séchoir - maturateur à lit fluidisé SCHUGI à 4 compartiments. Dans les 3 premiers compartiments, le produit granulé est séché par de l'air à 120-125°C, puis il est refroidi par de l'air à 25°C dans le dernier compartiment.

Le produit granulé, séché et refroidi est ensuite tamisé en continu sur un tamis rotatif équipé d'une toile métallique de 740 µm.

Le mannitol cristallin de départ E et le mannitol pulvérulent F conforme à l'invention préparé avec une solution de mannitol comme liant, présentent les caractéristiques rassemblées dans le tableau IV suivant.

**Tableau IV**

| Paramètres | E | F |
|---|---|---|
| Diamètre moyen laser (µm) | 90 | 101 |
| Densité tassée (g/ml) | 0,76 | 0,77 |
| Note d'écoulement (valeur/100) | 40 | 60 |
| Richesse en mannitol (% en poids) | > 98 | > 98 |
| Vitesse de dissolution dans l'eau (s) | 47 | 52 |

### EXEMPLE 5

D'autres produits pulvérulents conformes à l'invention sont préparés en appliquant le procédé décrit dans l'exemple 1, mais en modifiant les conditions de mise en oeuvre de manière à obtenir une gamme d'échantillons ayant une granulométrie, une densité tassée et un écoulement variables. Les produits obtenus présentent les caractéristiques énoncées dans le tableau V ci-dessous, et sont comparés à des mannitol pulvérulents connus par ailleurs.

**Tableau V**

| Paramètres | Produits conformes à l'invention | Produits réalisés par cristallisation | Produits réalisés par extrusion | Produits réalisés par atomisation |
|---|---|---|---|---|
| Diamètre moyen laser (µm) | 100 - 200 | 50 - 150 | 250 - 600 | 80 - 200 |
| Densité tassée (g/ml) | 0,7 - 0,8 | 0,75 - 0,8 | 0,65 - 0,75 | < 0,6 |
| Note d'écoulement (valeur/100) | 60 - 90 | < 60 | 60 - 80 | 70 - 90 |
| Richesse en mannitol (% en poids) | > 98 % | > 98 % | > 98 % | > 98 % |
| Vitesse de dissolution dans l'eau (s) | 20 - 60 | 15 - 25 | 60 - 80 | 8 - 15 |

Les mannitol pulvérulents conformes à l'invention possèdent tous, comparativement aux produits de l'art antérieur, d'excellentes propriétés fonctionnelles, d'aptitude à l'écoulement et de vitesse de dissolution dans l'eau pour une fine granulométrie et une densité tassée élevée et ceci avec une grande richesse en mannitol. Ces nouveaux produits, notamment ceux présentant une aptitude à l'écoulement importante pour une densité tassée supérieure à 0,7 g/ml, sont particulièrement aptes à être utilisés sans inconvénient dans les industries pharmaceutiques, en particulier comme excipients pour l'application remplissage de gélules. A la connaissance de la société Demanderesse, il n'existe pas de mannitol pulvérulent qui présente de telles propriétés.

### EXEMPLE 6

Il est procédé à la comparaison des propriétés pharmaceutiques des mannitol pulvérulents de l'invention (en l'espèce le mannitol pulvérulent B de l'exemple 1) et de l'art antérieur. Les propriétés pharmacotechniques ont été mesurées avec les méthodes de la pharmacopée européenne et figurent dans le tableau VI suivant. Les tailles des particules ont été déterminées par granulométrie laser.

**Tableau VI**

| | Produits réalisés par cristallisation | Produits réalisés par atomisation | Produits réalisés par extrusion | Mannitol pulvérulent B |
|---|---|---|---|---|
| Volume apparent (ml) Avant tassement V₀ | 162 | 208 | 152 | 182 |
| Après tassement V₁₀ | 156 | 206 | 148 | 167 |
| V₅₀₀ | 126 | 188 | 136 | 143 |
| V₁₂₅₀ | 124 | 186 | 135 | 142 |
| Aptitude au tassement V₁₀ - V₅₀₀ (ml) | 30 | 18 | 12 | 24 |
| Masse volumique apparente (g/ml) | | | | |
| Avant tassement | 0,617 | 0,481 | 0,658 | 0,549 |
| Après tassement | 0,806 | 0,538 | 0,741 | 0,704 |
| Ecoulement (s) | Infini | 7 | 9 | 5 |
| Taille des particules (µm) | 80 | 170 | 400 | 106 |

Le mannitol pulvérulent B conforme à l'invention possède les propriétés rhéologiques indispensables et recherchées dans de nombreux procédés galéniques, c'est-à-dire :
- d'écoulement libre, qui permet une homogénéité de poids du médicament et de dosage de l'actif,
- de densités libre et tassée élevées, qui permettent de réduire la taille du médicament final et en conséquence le rendront plus aisé à avaler,
- de granulométrie, compatible avec celles de la majorité des actifs, ce qui assure une homogénéité de répartition de l'actif et une dose exacte en actif dans le médicament.

On constate en effet que le mannitol cristallisé au contraire, ne s'écoule pas librement, et son utilisation imposerait une étape préalable de granulation.

Le mannitol poudre issu de procédé d'atomisation, possède quant à lui des densités libre et tassée plus faibles, ce qui donnera, dans les procédés galéniques à faible densification, des médicaments de taille plus élevée et donc plus difficile à avaler.

Enfin, le mannitol poudre réalisé par extrusion présente une taille de particules trop élevée par rapport à celle des principes actifs ; il sera utilisé plutôt dans des formulations où l'actif est granulé ou pelliculé.

### EXEMPLE 7

Il est procédé à la comparaison d'un mannitol pulvérulent conforme à l'invention (en l'espèce le mannitol pulvérulent B de l'exemple 1) et d'autres mannitol poudres dans la formulation d'une poudre de remplissage de gélules.

L'enveloppe de gélule est de taille 2, petite taille facile à avaler, et la masse de poudre est de 250 mg par gélule.

Le remplissage des gélules est semi-automatique, par arasement et vibrations.

Les propriétés pharmacotechniques recherchées sont habituellement, pour cette application:
- un écoulement libre pour remplissage uniforme de la gélule
- une densité tassée supérieure à 0,675 g/ml, permettant l'incorporation de 250 mg de poudre dans la gélule de taille 2 (volume interne de 0,37 ml)
- une granulométrie comprise entre 80 et 150 µm pour obtenir un mélange homogène avec l'actif.

Le tableau VII suivant présente la comparaison des propriétés pharmacotechniques recherchées.

**Tableau VII**

| Propriétés recherchées | Tests effectués | Produits réalisés par cristallisation | Produits réalisés par atomisation | Produits réalisés par extrusion | Mannitol pulvérulent B |
|---|---|---|---|---|---|
| Ecoulement libre | Ecoulement (s) | *Infini* | 7 | 9 | 5 |
| Densité tassée (> 0.675) | Masse volumique apparente après tassement V₁₂₅₀ (g/ml) | 0,806 | 0,538 | 0,741 | 0,704 |
| Granulométrie (80 - 150 µm) | Diamètre moyen laser (µm) | 80 | 170 | 400 | 106 |

Le mannitol pulvérulent B conforme à l'invention est le seul qui possède l'ensemble des propriétés recherchées, et donc le seul qui permette la formulation de cette poudre. pour gélules.

### EXEMPLE 8

Il est procédé à la comparaison d'un mannitol pulvérulent conforme à l'invention (en l'espèce le mannitol pulvérulent B de l'exemple 1) et d'autres mannitol poudres dans la formulation d'une poudre de remplissage de gélules.

L'enveloppe de gélules est de taille 0, taille permettant une dose élevée d'actifs, c'est-à-dire jusqu'à 700 mg.

Les gélules sont remplies par la technique du compresso-doseur.

Pour la formulation de la poudre de remplissage de gélules, le remplissage est simulé sur un appareil spécialement conçu, équipé d'une matrice de volume 0,78 ml, et d'un poinçon de diamètre 6,3 mm.

La partie inférieure de la matrice peut s'ouvrir et permet ainsi l'éjection de la carotte formée dans une enveloppe de gélule de taille 0.

Lors de l'essai, la poudre est comprimée dans la matrice à l'aide du poinçon jusqu'à l'obtention d'une carotte suffisamment cohésive pour être manipulée.

Il faut éviter une cohésion trop importante de cette carotte qui diminuerait sa vitesse de dissolution, car une dissolution rapide est nécessaire pour un effet thérapeutique immédiat du médicament.

Un lubrifiant, le stéarate de magnésium, est additionné à la poudre pour faciliter le glissement de la carotte lors de son éjection.

Sa teneur est dépendante de la poudre utilisée et est déterminée empiriquement.

Pour la sélection du mannitol-pulvérulent le mieux adapté à cette formulation de poudre pour gélule, les essais sont réalisés avec le mélange binaire mannitol pulvérulent et stéarate de magnésium.

Le tableau VIII suivant présente l'ensemble des résultats obtenus.

**Tableau VIII**

| | Produits réalisés par cristallisation | Produits réalisés par atomisation | Produits réalisés par extrusion | F |
|---|---|---|---|---|
| Taux de stéarate de magnésium (%) | 0,9 | 1,4 | 0,9 | 1 |
| Densité initiale de la poudre (g/ml) | 0,67 | 0,49 | 0,68 | 0,63 |
| Densité finale de la carotte (g/ml) | 0,99 | 0,84 | 1,05 | 1,09 |
| Poids de la carotte (mg) | 520 | 390 | 530 | 494 |
| Taille de la carotte (mm) | 16,89 | 14,85 | 16,12 | 14,48 |
| Temps de dissolution de la gélule (s) | 60 | 165 | 430 | 180 |
| Quantité maximale dans une gélule de taille 0 (mg) | 671 | 572 | 717 | 744 |

Le mannitol pulvérulent le mieux adapté est le mannitol selon l'invention, sur les critères respectés de :
- remplissage de la carotte uniforme durant les essais,
- densité finale élevée, supérieure à tous les autres mannitol pulvérulents,
- temps de dissolution faible (3 minutes).

Le mannitol cristallisé a généré en effet des problèmes de fluctuation de poids des carottes dus à son manque d'écoulement.

Le mannitol produit par atomisation a donné des carottes de densité trop faible : en le remplaçant par le mannitol pulvérulent de l'invention, il est possible de diminuer la taille de la gélule, ce qui améliore nettement le confort des patients.

Le mannitol fabriqué par extrusion a une granulométrie inadaptée. Outre que sa différence avec celle de l'actif créera des problèmes d'irrégularité de dosage, elle a aussi pour conséquence une dissolution trop lente de la gélule.

Seul le mannitol pulvérulent de l'invention permettra la formulation de la poudre pour remplissage de gélules en respectant l'ensemble des critères fixés.

## Revendications

1. Mannitol pulvérulent **caractérisé en ce qu'**il présente :
- un diamètre moyen compris entre 60 et 200 µm, de préférence compris entre 80 et 180 µm,
- une densité tassée, déterminée selon un test A selon la méthode préconisée dans le mode d'emploi du testeur poudre HOSOKAWA de type P.T.N., comprise entre 0,65 et 0,85 g/ml, de préférence comprise entre 0,7 et 0,8 g/ml,
- une note d'écoulement d'au moins 60, de préférence comprise entre 60 et 90, utilisant l'appareil POWDER TESTER comercialisé par la société HOSOKAWA.

2. Mannitol pulvérulent selon la revendication 1, **caractérise en ce qu'**il présente une richesse en mannitol au moins égale à 96% en poids, de préférence au moins égale à 98% en poids.

3. Mannitol pulvérulent selon l'une ou l'autre des revendications 1 et 2, **caractérisé en ce qu'**il présente une vitesse de dissolution dans l'eau selon un test B compris entre 20 et 60 s.

4. Procédé de préparation d'un mannitol pulvérulent selon l'une quelconque des revendications 1 à 3, **caractérisé en ce qu'**il comprend une étape de granulation d'un mannitol poudre, obtenu par cristallisation dans l'eau ou dans un autre solvant comme l'alcool, par voie humide à l'aide d'un liant et une étape de maturation, par séchage, du mannitol pulvérulent ainsi obtenu.

5. Procédé de préparation selon la revendication 4, **caractérisé en ce que** l'étape de granulation est réalisée dans un mélangeur granulateur continu.

6. Utilisation du mannitol pulvérulent selon l'une quelconque des revendications 1 à 3 ou obtenu par le procédé selon l'une ou l'autre des revendications 4 et 5 en tant qu'excipient dans des compositions destinées en particulier au domaine pharmaceutique.

7. Utilisation du mannitol pulvérulent selon l'une quelconque des revendications 1 à 3 ou obtenu par le procédé selon l'une ou l'autre des revendications 4 et 5 en tant qu'excipient pour poudre de remplissage de gélules.

## Patentansprüche

1. Pulverförmiges Mannit, **dadurch gekennzeichnet, dass** es aufweist:
- einen mittleren Durchmesser umfasst zwischen 60 und 200 µm, vorzugsweise umfasst zwischen 80 und 180 µm.
- eine Rütteldichte, die gemäß einem Test A gemäß der Methode bestimmt wird, die in der Bedienungsanleitung des Pudertesters vom Typ P.T.N von HOSOKAWA empfohlen wird, umfasst zwischen 0,65 und 0,85 g/ml, vorzugsweise umfasst zwischen 0,7 und 0,8 g/ml.
- einen Fließwert von wenigstens 60, vorzugsweise umfasst zwischen 60 und 90 unter Verwendung eines Pudertestgerätes, das durch die HOSOKAWA-Gesellschaft vertrieben wird.

2. Pulverförmiges Mannit gemäß Anspruch 1, **dadurch gekennzeichnet, dass** es einen Mannitgehalt von wenigstens gleich 96 Gew.-% aufweist, vorzugsweise wenigstens gleich 98 Gew.-%.

3. Pulverförmiges Mannit gemäß einem der Ansprüche 1 und 2, **dadurch gekennzeichnet, dass** es eine Lösungsgeschwindigkeit in Wasser gemäß einem Test B, umfasst zwischen 20 und 60 s, aufweist.

4. Verfahren zur Herstellung eines pulverförmigen Mannits **gemäß** einem beliebigen der Ansprüche 1 bis 3, **dadurch gekennzeichnet, dass** es einen Granulierungsschritt von puderförmigem Mannit, das durch Kristallisation in Wasser oder einem anderen Lösungsmittel wie Alkohol erhalten wurde, mittels Feuchtigkeit unter Zuhilfenahme eines Bindemittels und einen Reifungsschritt durch Trocknen des auf diese Weise erhaltenen pulverförmigen Mannits umfasst.

5. Herstellungsverfahren gemäß Anspruch 4, **dadurch gekennzeichnet, dass** der Granulierungsschritt in einem kontinuierlichen Mischer-Granulierer ausgeführt wird.

6. Verwendung des pulverförmigen Mannits gemäß einem beliebigen der Ansprüche 1 bis 3 oder erhalten durch das Verfahren gemäß einem der Ansprüche 4 und 5 als Triebstoff in Zusammensetzungen, die insbesondere für das Gebiet der Pharmazie bestimmt sind.

7. Verwendung des pulverförmigen Mannits gemäß einem beliebigen der Ansprüche 1 bis 3 oder erhalten durch das Verfahren gemäß einem der Ansprüche 4 und 5 als Trägerstoff für Füllpuder von Gelatinekapseln.

## Claims

1. Pulverulent mannitol having:
- an average diameter of between 60 and 200 µm, preferably of between 80 and 180 µm,
- a packed density, determined according to a Test A, according to the method recommended by the operating instructions of the powder tester of the PT-N model of between 0.65 and 0.85 g/ml, preferably of between 0.7 and 0.8 g/ml,
- a flow factor of at least 60, preferably of between 60 and 90, using the POWDER TESTER apparatus marketed by the HOSOKAWA Company.

2. Pulverulent mannitol according to Claim 1 **characterized in that** it has a mannitol content at least equal to 96% by weight, preferably at least equal to 98% by weight.

3. Pulverulent mannitol according to one or other of the Claims 1 and 2, **characterized in that** it has a rate of dissolution in water according to a Test B of between 20 and 60 seconds.

4. Process for preparing pulverulent mannitol according to any one of the Claims 1 to 3, **characterized in that** it comprises a step of granulating a mannitol powder obtained by crystallization in water or another solvent such as alcohol by a wet route with the aid of a binder and a maturing step, by drying, of the pulverulent mannitol thus obtained.

5. Preparation process according to Claim 4, **characterized in that** the granulation stage is carried out in a continuous mixer granulator.

6. Use of pulverulent mannitol according to any one of the Claims 1 to 3 or obtained by the process according to one or other of the Claims 4 and 5 as an excipient in preparations intended in particular for the pharmaceutical field.

7. Use of pulverulent mannitol according to any one of the Claims 1 to 3 or obtained by the process according to one or other of the Claims 4 and 5 as an excipient for powder for filling hard capsules.
